(19) Europäisches Patentamt
European Patent Office
Office européen des brevets

(11) Publication number: **0 048 045**
**A1**

(12) # EUROPEAN PATENT APPLICATION

(21) Application number: 81200929.8

(22) Date of filing: 25.08.81

(51) Int. Cl.[3]: C 07 D 295/12
C 07 D 213/56, C 07 D 333/24
A 61 K 31/495

(30) Priority: 12.09.80 NL 8005133

(43) Date of publication of application: 24.03.82 Bulletin 82/12

(84) Designated Contracting States: AT BE CH DE FR GB IT LI LU NL SE

(71) Applicant: DUPHAR INTERNATIONAL RESEARCH B.V
C.J. van Houtenlaan 36
1381 CP Weesp(NL)

(72) Inventor: Haeck, Hans Heinz
c/o OCTROOIBUREAU ZOAN B.V. Apollolaan 151
NL-1077 AR Amsterdam(NL)

(72) Inventor: Hillen, Feddo Cornelius
c/o OCTROOIBUREAU ZOAN B.V. Appollolaan 151
NL-1077 AR Amsterdam(NL)

(74) Representative: Muis, Maarten, Drs. et al,
OCTROOIBUREAU ZOAN B.V. Apollolaan 151
NL-1077 AR Amsterdam(NL)

(54) **Phenyl piperazine derivatives having antiagressive activity.**

(57) It has been found that the group of new phenyl piperazine derivatives of formula 3 of the formula sheet and the salts thereof have a strong and selective antiagressive activity. The compounds can be prepared in a manner known for analogous compounds and be processed to the usual compositions.

EP 0 048 045 A1

Phenyl piperazine derivatives having antiagressive activity

The invention relates to new phenyl piperazine derivatives, to the preparation of these compounds, and to compositions comprising at least one of these compounds as an active substance.

Various phenyl piperazine derivatives having a variety of pharmacological properties are known.

United States Patent Specification 2,722,529, for example, relates to compounds of formula 1 of the formula sheet, in which B is a straight or branched alkyl group having 2-6 carbon atoms and Q is an acyl group, sulphonyl group or carbamoyl group. These compounds are said to have a strong sympatholytic activity and sometimes a hypotensive activity.

From United States Patent Specification 2,833,770 is furthermore known a group of compounds of the general formula 2 of the formula sheet, in which formula R is a chlorine atom or a bromine atom, R' is hydrogen or a methyl group and m is an integer number from 2-5. Of these compounds a sympatholytic activity is also known.

It has been found that phenyl piperazine derivatives of formula 3 of the formula sheet, in which $R_1$ is a hydrogen atom, a methyl group or a substituted or non-substituted phenyl group, $R_2$ is a hydrogen atom or a methyl group, or $R_1$ and $R_2$ together with the carbon atom to which they are bound are a cyclic alkyl group having 3-7 C-atoms, $R_3$ is a hydrogen atom or an alkyl group having 1 or 2 carbon atoms, $R_4$ is a trifluoromethyl group, A is a straight or branched alkyl group having 1-4 C-atoms, and Z is a cycloalkyl group having 5-7 C-atoms, a phenyl group or a phenyl group substituted by 1, 2 or 3 substituents selected from the group consisting of alkyl, alkoxy, halogen and $CF_3$, a furanyl group, a pyridyl group or a pyrimidyl group, an optionally substituted benzyl, aryloxy, arylthio, arylsulfonyl, arylsulfinyl or heteroaryloxy group and the salts thereof

have a strong antiagressive activity. Some of the compounds belonging to this group moreover have some analgetic activity.

Surprisingly, this antiagressive activity of the compounds according to the invention proved not to be associated with the sympatholytic properties described for the above-mentioned known and structurally closely related compounds, which properties are undesired for antiagressive agents.

Other undesired side effects, such as dopaminolytic, muscle relaxing and sedative properties which are undesired for use as antiagressive agents are also absent in the dosages in which the antiagressive activity occurs.

It is extremely surprising that the compounds according to the invention show a strong and particularly selective antiagressive activity, since structurally very closely related known compounds have a quite different pharmacological activity pattern.

The activity of the compounds ($ED_{50}$-value) was determined in a test on antiagressive activity in isolated mice (Advances in Pharmacol. 5, (1967), 79). In this test, male albino mice were kept isolated for a period of 4 weeks and then selected for the test on the basis of fighting behaviour present. The selection criterion is the occurrence of 3 or more fighting periods within 3 minutes after a mouse not kept isolated was placed in the cage of the mouse which had been kept isolated.

The compounds to be examined were administered orally to the selected mice. Per dose 5 mice were used. Sixty minutes after administration of the compounds to be tested, the animals were again evaluated for fighting behaviour. The compound to be examined is inactive in the dose administered when this time also 3 or more fighting periods were observed within 3 minutes after a mouse not kept isolated was placed in the case of the mouse which had been kept isolated. From the results obtained the $ED_{50}$-value in mg of active substance per kg of body weight was calculated. The $ED_{50}$-values of the compounds according to the invention is smaller than 10 mg/kg and in most of the cases is between 1 and 5 mg/kg.

Since in the compounds according to the invention the sympatholytic activity and also other undesired side effects mentioned of the known compounds were in addition not found, the new compounds are excellently suitable, due to their surprising selective antiagressive activity pattern, for being used in the treatment of intrapunitive and extrapunitive behaviour and overt agressive behaviour in man and animal.

For medical use in men is to be considered first of all the control of agressive symptoms in psychiatric illnesses and serious forms of psychopathological agression.

As an application possibility in the veterinary field are to be considered in particular those forms of agression which occur in transporting agricultural domestic animals and the mixing of groups of these animals.

The quantity, frequency and way of administration may differ for each individual case; also dependent on the nature and the severity of the disturbances. In general a dose of 5-500 mg and preferably 25-150 mg per day will be suitable for humane application.

For veterinary purposes the dose is preferably 0.1-10 mg/kg of body weight.

The active compounds according to the invention and their salts can be processed to compositions such as pills, tablets, coated tablets, capsules, powders, injection liquids and the like according to known standard techniques, while using the conventional auxiliary substances such as solid and liquid carrier materials.

As examples of pharmaceutically acceptable acids with which the compounds according to the invention can form salts may be mentioned hydrochloric acid, sulphuric acid, nitric acid, citric acid, fumaric acid, maleic acid, tartaric acid, methanesulfonic acid, benzoic acid and the like.

The compounds of formula 3 and their salts can be prepared according to methods suitable for the synthesis of analogous compounds. The invention therefore also relates to the preparation of the new compounds and the salts thereof.

Dependent on the symbols of $R_1$-$R_4$, A and Z, the compounds of the general formula 3 can be obtained according to at least one of the methods below:

a) Reaction of a compound of formula 4, in which $R_3$, $R_4$ and A have the above-mentioned meanings, with an ester (preferably the methyl ester of ethyl ester) of a compound of formula 5, in which $R_1$, $R_2$ and Z have the above-mentioned meanings.
The reaction is preferably carried out with an excess of the ester, in a suitable solvent, at a temperature between room temperature and the boiling point of the solvent used (see J. Pharm. Soc. Japan, 62 (1942), 531).

b) Reaction of a compound of formula 4 with an acid halide of formula 6 or the mixed anhydride of formula 7, or with the ester of formula 8 (so-called Mukayama ester; see Chem. Letters, 1163, (1975)).
This reaction is carried out in an organic solvent, for example, toluene, dioxan, ether, methylene chloride or tetrahydrofuran, at temperatures between room temperature and the boiling point of the solvent used.

The compounds according to the invention can also be obtained by reaction of a compound of formula 9 with a compound of formula 10. In these formulae, $R_1$-$R_4$, A and Z have the above-mentioned meanings and X is a so-called leaving group, preferably chlorine, bromine or tosylate. The reaction may be carried out both with and without an inert organic solvent. Suitable solvents are, for example, methyl ethyl ketone, dimethyl formamide, tetrahydrofuran, petroleum ether, alcohol and acetonitrile. As an acid binder is preferably used $NaHCO_3$ or potassium carbonate. The reaction temperature is usually between room temperature and the reflux temperature of the solvent used, and the reaction duration varies from a few hours to approximately 20 hours.

Compounds according to the invention of formula 3, in which $R_3$ is an alkyl group and $R_1$, $R_2$, $R_4$, A and Z have the above-mentioned meanings, may also be prepared by reaction of a compound of formula 3 not substituted at the nitrogen atom with a compound $R_3Y$, in which $R_3$ is alkyl and Y is halogen (preferably iodine) or $(SO_4)_{\frac{1}{2}}$.

This reaction is usually carried out in a solvent , for example, benzene, DMSO or dimethyl formamide in

the presence of a strong base such as, for example, KOH or NaH in anhydrous circumstances at temperatures between room temperature and the reflux temperature of the solvent (see J. Org. Chem. 14 (1949), 1099).

In similar reaction circumstances, compounds of formula 3 can be prepared by reaction of a compound of formula 11 with a compound of formula 12, in which formulae $R_1$-$R_4$, A, Z and X have the above-mentioned meanings.

Compounds according to the invention of formula 3, in which $R_4$ is trifluoromethyl, can be obtained by reaction of a compound of formula 13, in which $R_4$ is trifluoromethyl and Hal is halogen, preferably bromine, with a compound of formula 14, in which $R_1$-$R_3$, A and Z have the above-mentioned meanings.

This reaction is carried out in a solvent, for example, DMSO, in the presence of an acid binder, for example $Na_2CO_3$. The reaction mixture is stirred at elevated temperature (100-180°C) for a few hours (see German Offenlegungsschrift 2,024,826).

The compounds of formula 3 can furthermore be prepared by converting a compound of formula 15, in which $R_1$-$R_4$, A and Z have the above-mentioned meanings, with 1,2--dibromoethane. The reaction is carried out in an organic solvent such as dioxan or butanol in the presence of an acid binder, for example, $K_2CO_3$, at a temperature of 20-120°C (see Arzneimittel Chemie 27 (11), (1977), 2077-2086).

The compounds of formula 3 can also be obtained by converting a compound of formula 16 with a compound of formula 17, in which formulae $R_1$-$R_4$, A and Z have the above-mentioned meanings. This conversion is preferably carried out in a solvent, for example butanol, in the presence of an acid binder, for example $K_2CO_3$, at temperatures between room temperature and the boiling-point of the solvent used (see British Patent Specification 943,739).

Furthermore, the compounds of formula 3 can be prepared in similar reaction circumstances by reaction of a compound of formula 18 with a compound of formula 19, in which formula $R_1$-$R_4$, A and Z have the above-mentioned meanings

(see Coll. Czech. Chem. Comm. 6 (1934), 211).

Compounds of the general formula 3 in which A is the group $-CH_2-CH_2-$, can be prepared by converting a compound of formula 9 with a compound of formula 20, in which formula $R_1$-$R_4$ and Z have the above-mentioned meanings, The two reaction components are heated at 100°C for 1-6 hours preferably without a solvent. However, the starting substances can also be reacted in a solvent such as acetone, methyl ethyl ketone or toluene at temperatures between room temperature and the boiling-point of the solvent used.

The compounds of the general formula 3, in which $R_1$-$R_4$ and Z have the above-mentioned meanings and A is the methylene group, can also be obtained by reacting a compound of formula 9 with a compound of formula 12 in the presence of a formaline solution in alcohol, at temperatures between room temperature and the boiling-point of the solvent.

The invention will now be described in greater detail with reference to the ensuing specific examples.

EXAMPLE I

N-[2-{4-(3-trifluoromethylphenyl)-1-piperazinyl}ethyl]1-phenyl cyclohexane carboxamide.HCl

10 Mmol (2.73 g) of 2-[4-(3-trifluoromethylphenyl)-1-piperazinyl]ethylamine were dissolved in 20 ml of methylenechloride. While stirring, a solution of 11 mmol (2.45 g) of 1-phenylcyclohexane carbonyl chloride in 10 ml of methylenechloride was added dropwise.

After leaving to stand at room temperature for 12 hours, the reaction mixture was evaporated to dryness in vacuo and the residue was recrystallized from methylenechloride/ether (1:1). The material thus obtained was recrystallized once again from ethyl acetate, after which the title compound was obtained having a melting-point of 149-150°C.

In an analogous manner the following compounds were prepared from the said starting substances:

1) N-[2-{4-(3-trifluoromethylphenyl)-1-piperazinyl}ethyl]-1-phenyl cyclopropane carboxamide.HCl, melting-point 176.5-178.5°C, from 2-[4-(3-trifluoromethylphenyl)-1-piperazinyl] ethylamine and 1-phenyl cyclopropane carbonylchloride.

2) N-[2-{4-(3-trifluoromethylphenyl)-1-piperazinyl}ethyl]N-methyl 1-phenylcyclohexane carboxamide.HCl, melting-point 188-190°C from 2-[4-(3-trifluoromethylphenyl)-1-piperazinyl]ethylmethylamine and 1-phenyl cyclohexane carbonyl chloride.

3) N-[2-{4-(3-trifluoromethylphenyl)-1-piperazinyl}ethyl] 1-(4--chlorophenyl)cyclohexane carboxamide.HCl, melting-point 134.5-136.5°C, from 2-[4-(3-trifluoromethylphenyl)-1-piperazinyl]ethylamine and 1-(4-chlorophenyl)cyclohexane carbonyl chloride.

4) N-[2-{4-(3-trifluoromethylphenyl)-1-piperazinyl}-1-methylethyl]-1-phenyl cyclohexane carboxamide.HCl, melting-point 120-129°C, from 1-[4-(3-trifluoromethylphenyl)-1-piperazinyl]-2-propylamine and 1-phenyl cyclohexane carbonyl chloride.

5) N-[2-{4-(3-trifluoromethylphenyl)-1-piperazinyl}ethyl]diphenylacetamide.HCl, melting-point 103-105°C, from 2-[4-(3--trifluoromethylphenyl)-1-piperazinyl]ethylamine and diphenylacetyl chloride.

6) N-[2-{4-(3-trifluoromethylphenyl)-1-piperazinyl}ethyl]thienylacetamide.HCl, melting-point 143-144.5°C from 2-[4-(3--trifluoromethylphenyl)-1-piperazinyl]ethylamine and thienylacetyl chloride.

7) N-[2-{4-(3-trifluoromethylphenyl)-1-piperazinyl}ethyl]4--chlorophenoxy acetamide.HCl melting-point 175.5-177°C, from 2-[4-(3-trifluoromethylphenyl)-1-piperazinyl]ethylamine and 4-chlorophenoxy acetyl chloride.

8) N-[2-{4-(3-trifluoromethylphenyl)-1-piperazinyl}ethyl]thiophenoxy acetamide, melting-point 183-184°C, from 2-[4-(3--trifluoromethylphenyl)-1-piperazinyl]ethylamine and thiophenoxy acetyl chloride.

9) N-[2-{4-(3-trifluoromethylphenyl)-1-piperazinyl}ethyl]phenyl sulfonyl acetamide.HCl, melting-point 220-223°C, from 2-[4-(3--trifluoromethylphenyl)-1-piperazinyl]ethylamine and phenyl sulfonyl acetyl chloride.

10) N-[2-{4-(3-trifluoromethylphenyl)-1-piperazinyl}ethyl]cyclohexyl acetamine,HCl, melting-point 182.5-183.5°C, from 2-[4-(3-trifluoromethylphenyl)-1-piperazinyl]ethylamine and cyclohexyl acetyl chloride.

EXAMPLE II

N-[2-{4-(3-trifluoromethylphenyl)-1-piperazinyl}ethyl]dimethyl 4-methoxyphenyl acetamide.HCl

25 Mmol (7.7 g) of 2-[4-(3-trifluoromethylphenyl)-1-piperazinyl]ethylamine.HCl were suspended in 10 ml of toluene and 13.2 ml of a 20% $Na_2CO_3$ solution in water were then added. The mixture was cooled to 0°C after which a solution of 25 mmol (5.3 g) of dimethyl-4-methoxyphenyl acetyl chloride in 5 ml of toluene was added dropwise while stirring in approximately 20 minutes. The reaction mixture was then stirred at 0°C for another 45 minutes, after which it was brought at room temperature, at which temperature stirring was continued for another 2 hours. Ether and water were then added. The organic phase was separated and the aqueous layer was extracted twice with ether. The combined organic phase was washed once with 5% $NaHCO_3$ solution and then dried on $MgSO_4$. After evaporating the solvents the residue was purified chromatographically over silica gel with ethyl acetate as an eluent. The free base thus obtained was taken up in 10 ml of ethanol and 1 equivalent of hydrochloric acid in ethanol was added. The clear solution was then evaporated under reduced pressure and the residue was recrystallized from isopropanol /ether. The melting-point of the title compound obtained in this manner was 148-149.5°C.

The following compounds were obtained in an identical manner:

1) N-[2-{4-(3-trifluoromethylphenyl)-1-piperazinyl}ethyl]3--trifluoromethylphenyl acetamide.HCl, melting-point 138--141°C, from 2-[4-(3-trifluoromethylphenyl)-1-piperazinyl] ethylamine and 3-trifluoromethylphenyl acetyl chloride.

2) N-[2-{4-(3-trifluoromethylphenyl)-1-piperazinyl}-2-methylethyl]-1-phenylcyclohexane carboxamide.maleate, melting-point 166°C, from 2-[4-(3-trifluoromethylphenyl)-1-piperazinyl] propylamine and 1-phenylcyclohexane carbonyl chloride.

3) N-[3-{4-(3-trifluoromethylphenyl)-1-piperazinyl} propyl]-1--phenylcyclohexane carboxamide, melting-point 120-129°C from 3-[4-(3-trifluoromethylphenyl)-1-piperazinyl]propylamine and 1-phenylcyclohexane carbonyl chloride.

4) N-[2-{4-(3-trifluoromethylphenyl)-2-piperazinyl}ethyl]dimethyl 4-fluorophenyl acetamide, meltingpoint 89-90°C from 2-[4-(3-trifluoromethylphenyl)-1-piperazinyl]ethylamine and dimethyl-4-fluorophenylacetyl chloride.

5) N-[2-{4-(3-trifluoromethylphenyl)-1-piperazinyl}ethyl]-4--methoxyphenyl acetamide, melting-point 99-101°C from 2-[4-(3-trifluoromethylphenyl)-1-piperazinyl]ethylamine and 4-methoxyphenyl acetyl chloride.

6) N-[2-{4-(3-trifluoromethylphenyl)-1-piperazinyl}ethyl]-4--fluorophenyl acetamide, melting-point 95°C from 2-[4-(3--trifluoromethylphenyl)-1-piperazinyl]ethylamine and 4-fluorophenyl acetyl chloride.

7) N-[2-{4-(3-trifluoromethylphenyl)-1-piperazinyl}ethyl]2,6--dichlorophenyl acetamide, melting-point 156°C from 2-[4-(3--trifluoromethylphenyl)-1-piperazinyl]ethylamine and 2,6--dichlorophenyl acetyl chloride.

8) N-[2-{4-(3-trifluoromethylphenyl)-1-piperazinyl}ethyl]dimethyl 3-trifluoromethylphenyl acetamide.maleate, melting-point 108-109°C from 1-[4-(3-trifluoromethylphenyl)-1-piperazinyl]ethylamine and dimethyl 3-trifluoromethylphenyl acetyl chloride.

9) N-[2-{4-(3-trifluoromethylphenyl)-1-piperazinyl}ethyl]4--trifluoromethylphenyl acetamide, melting-point 93-96°C, from 2-[4-(3-trifluoromethylphenyl)-1-piperazinyl]ethylamine and 4-trifluoromethylphenyl acetyl chloride.

10) N-[2-{4-(3-trifluoromethylphenyl)-1-piperazinyl}ethyl]dimethyl 4-trifluoromethylphenyl acetamide from 2-[4-(3-trifluoromethylphenyl)-1-piperazinyl]ethylamine and dimethyl 4-trifluoromethylphenyl acetyl chloride.

11) N-[2-{4-(3-trifluoromethylphenyl)-1-piperazinyl}ethyl]phenoxy acetamide.$CH_3SO_3H$, melting-point 145.5-147.5°C, from 2-[4-(3-trifluoromethylphenyl)-1-piperazinyl]ethylamine and phenoxyacetyl chloride.

12) N-[2-{4-(3-trifluoromethylphenyl)-1-piperazinyl}ethyl]bis-4-chlorophenoxy acetamide.maleate, melting-point 182.5-183.5°C, from 2-[4-(3-trifluoromethylphenyl)-1-piperazinyl]ethylamine and bis-4-chlorophenoxy acetyl chloride.

EXAMPLE III

N-[2-{4-(3-trifluoromethylphenyl)-1-piperazinyl}ethyl]dimethylphenyl acetamide.HCl

10 Mmol (2.73 g) of 2-[4-(3-trifluoromethylphenyl)-1-piperazinyl]ethylamine were dissolved in 20 ml of methylenechloride. A solution of 11 mmol ( 2 g) of dimethyl phenyl acetyl chloride in 10 ml of methylene chloride was added dropwise while stirring. After leaving to stand at room temperature for 6 hours, 15 ml of 2 N KOH were added, the organic phase was separated and the aqueous layer was extracted 2 times with methylene chloride. The collected organic phase was washed twice with water and then dried on $K_2CO_3$. The solution thus obtained was evaporated under reduced pressure and the remaining residue was taken up in 15 ml of ethanol. After adding 1 equivalent hydrochloric acid in alcohol, evaporation was carried out again under reduced pressure, and the salt thus obtained was recrystallized from ethanol/ether. The title compound obtained in this manner had a melting-point of 134-136.5°C.

In the same manner the following compounds were prepared:

1) N-[2-{4-(3-trifluoromethylphenyl)-1-piperazinyl}ethyl]1-(4-methylphenyl)cyclopentane carbonamide.HCl, melting-point 186-188°C, from 2-[4-(3-trifluoromethylphenyl)-1-piperazinyl]ethylamine and 1-(4-methylphenyl)cyclopentane carbonyl chloride.

2) N-[4-{4-(3-trifluoromethylphenyl)-1-piperazinyl}butyl]-1--phenylcyclohexane carbonamide.oxalate, melting-point 81.5-86°C, from 4-[4-(3-trifluoromethylphenyl)-1-piperazinyl]butylamine and 1-phenylcyclohexane carbonyl chloride.

3) N-[2-{4-(3-trifluoromethylphenyl)-1-piperazinyl}ethyl]phenyl acetamide, melting-point 85-86°C, from 2-[4-(3-trifluoromethylphenyl)-1-piperazinyl]ethylamine and phenyl acetyl chloride.

4) N-[2-{4-(3-trifluoromethylphenyl)-1-piperazinyl}ethyl]phenyl methyl acetamide, melting-point 72-73°C, from 2-[4-(3--trifluoromethylphenyl)-1-piperazinyl]ethylamine and phenyl methyl acetyl chloride.

EXAMPLE IV

N-[2-{4-(3-trifluoromethylphenyl)-1-piperazinyl}ethyl]cyclohexyl acetamide

10 Mmol (1.42 g) of cyclohexyl acetic acid and 10 mmol (1.4 ml) of triethyl amine were dissolved in 25 ml of dry dioxan. A solution of 10 mmol (0.96 ml) of chloroformic acid ethyl ester in 5 ml of dry dioxan was then added dropwise at a temperature between 15 and 20°C. After stirring at room temperature for 1 hour a solution 10 mmol (2.73 g) of 2-[4-(3--trifluoromethylphenyl)-1-piperazinyl]ethylamine in 10 ml of dry dioxan was added slowly, the temperature being kept below 30°C. The reaction mixture was stirred for another hour, after which the precipitate formed was sucked off. The filtrate was evaporated under reduced pressure and the residue was recrystallized from cyclohexane. The material thus obtained was recrystallized again from cyclohexane-ether, after which the title compound was isolated. It had a melting-point of 130-131°C.

The following compounds were obtained in an identical manner:

1) N-[2-{4-(3-trifluoromethylphenyl)-1-piperazinyl}ethyl] (2-pyridyl)acetamide, melting-point 102.5-105.5°C, from 2-[4-(3-trifluoromethylphenyl)-1-piperazinyl]ethylamine and 2-pyridyl acetic acid.

2) N-[2-{4-(3-trifluoromethylphenyl)-1-piperazinyl}ethyl](3-pyridyl) acetamide.HCl, melting-point 153.5-154°C, from 2-[4-(3-trifluoromethylphenyl)-1-piperazinyl]ethylamine and 3-pyridyl acetic acid.

CLAIMS:

1. Compounds of formula 3 of the formula sheet and salts thereof with pharmaceutically acceptable acids, in which formula $R_1$ is hydrogen, methyl or a optionally substituted phenyl group,

$R_2$ is hydrogen or a methyl group or $R_1$ and $R_2$ together with the carbon atom to which they are bound are a cyclic alkyl group having 3-7 C-atoms,

$R_3$ is a hydrogen atom, a methyl group or an ethyl group,

$R_4$ is a trifluoromethyl group,

A is a straight or a branched alkyl group having 1-4 C-atoms, and

Z is an alkyl group having 5-7 C-atoms, a phenyl group or a phenyl group substituted by 1, 2 or 3 substituents selected from the group consisting of alkyl, alkoxy, halogen and $CF_3$, a furanyl group, a pyridyl group or a pyrimidyl group, an optionally substituted benzyl, aryloxy, arylthio, arylsulfonyl, arylsulfinyl or heteroaryloxy group.

2. N-[2-{4-(3-trifluoromethylphenyl)-1-piperazinyl}ethyl] phenoxy acetamide.

3. Pharmaceutical compositions, characterized in that as an active substance they comprise at least one compound of formula 3 of the formula sheet, in which $R_1$-$R_4$, A and Z have the meanings given in Claim 1, or a pharmaceutically acceptable acid addition salt thereof.

4. A method of preparing pharmaceutically active phenyl piperazine derivatives, characterized in that antiagressive active compounds of formula 3 of the formula sheet, in which $R_1$-$R_4$, A and Z have the meanings mentioned in Claim 1, and salts thereof with pharmaceutically acceptable acids are prepared in a manner known for the synthesis of analogous compounds.

5. A method as claimed in claim 4, characterized in that a compound of formula 4 of the formula sheet is converted with a) an ester of a compound of formula 5, or

b) with an acid halide of formula 6, a mixed anhydride of formula 7 or an ester of formula 8.

6. A method as claimed in claim 4, characterized in that a compound of formula 9 is converted with a compound of formula 10, in which $R_1$-$R_4$, A and Z have the meanings given in Claim 1 and X is a leaving group, such as chlorine, bromine or tosylate.

7. A method as claimed in Claim 4, characterized in that a compound of formula 3 in which $R_3$ is an alkyl group, is prepared by converting a compound of formula 3 not-substituted at the nitrogen atom, with a compound $R_3Y$, in which $R_3$ has the above-mentioned meaning and Y is halogen or the group $(SO_4)_{\frac{1}{2}}$.

8. A method as olaimed in Clam 4, characterized in that a compound of formula 11 is converted with a compound of formula 12, in which $R_1$-$R_4$, A and Z have the meanings given in Claim1 and X has the meaning given in Claim 6.

9. A method as claimed in Claim 4, characterized in that a compound of formula 13 in which Hal is a halogen atom and $R_4$ is trifluoromethyl is converted with a compound of formula 14 in which $R_1$-$R_3$, A and Z have the meanings given in Claim 1.

10. A method as claimed in Claim 4, characterized in that a compound of formula 15 in which $R_1$-$R_4$, A and Z have the meanings given in Claim 1 is converted with 1,2-dibromoethane.

11. A method as claimed in Claim 4, characterized in that a compound of formula 16 is converted with a compound of formula 17, in which $R_1$-$R_4$, A and Z have the meanings given in Claim 1.

12. A method as claimed in Claim 4, characterized in that a compound of formula 18 is converted with a compound of formula 19, in which $R_1$-$R_4$ and Z have the meanings given in Claim 1 and A is the group $-CH_2-CH_2-$.

13. A method as claimed in Claim 4, characterized in that a compound of formula 9 is converted with a compound of formula 20, in which $R_1$-$R_4$, A and Z have the meanings given in Claim 1.

14. A method as claimed in Claim 4, characterized in that a compound of formula 9 is converted with a compound of formula 12 in the presence of formaldehyde, in which

formulae A is the methylene group and $R_1$-$R_4$ and Z have the meanings given in Claim 1.

15. A method of controlling intrapunitive and extrapunitive behaviour and overt agressive behaviour in man and animal, characterized in that an active quantity of a compound of formula 3, in which $R_1$-$R_4$, A and Z have the meanings mentioned in Claim 1, or a salt thereof with a pharmaceutically acceptable acid, is administered.

16. A method of preparing pharmaceutical compositions, characterized in that a compound of Claim 1 is brought into a form suitable for administration.

CLAIMS FOR AUSTRIA:

1. A method of preparing pharmaceutically active phenyl piperazine derivatives, characterized in that antiagressively active compounds of formula 3 of the formula sheet, in which $R_1$ is hydrogen, methyl or a optionally substituted phenyl group,

$R_2$ is hydrogen or a methyl gorup or $R_1$ and $R_2$ together with the carbon atom to which they are bound are a cyclic alkyl group having 3-7 C-atoms,

$R_3$ is a hydrogen atom, a methyl group or an ethyl group,

$R_4$ is a trifluoromethyl group,

A is a straight or a branched alkyl group having 1-4 C-atoms, and

Z is an alkyl group having 5-7 C-atoms, a phenyl group or a phenyl group substituted by 1, 2 or 3 substituents selected from the group consisting of alkyl, alkoxy, halogen and $CF_3$, a furanyl group, a pyridyl group or a pyrimidyl group, an optionally substituted benzyl, aryloxy, arylthio, arylsulfonyl, arylsulfinyl or heteroaryloxy group,

and salts thereof with pharmaceutically acceptable acids are prepared in a manner known for the synthesis of analogous compounds.

2. A method as claimed in Claim 1, characterized in that a compound of formula 4 of the formula sheet is converted with a) an ester of a compound of formula 5, or

b) with an acid halide of formula 6, a mixed anhydride of formula 7 or an ester of formula 8.

3. A method as claimed in claim 1, characterized in that a compound of formula 9 is converted with a compound of formula 10, in which $R_1$-$R_4$, A and Z have the meanings given in Claim 1 and X is a leaving group, such as chlorine, bromine or tosylate.

4. A method as claimed in claim 1, characterized in that a compound of formula 3 in which $R_3$ is an alkyl group, is prepared by converting a compound of formula 3 not-substituted at the nitrogen atom, with a compound $R_3Y$, in

which $R_3$ has the above-mentioned meaning and Y is halogen or the group $(SO_4)_{\frac{1}{2}}$.

5. A method as claimed in Claim 1, characterized in that a compound of formula 11 is converted with a compound of formula 12, in which $R_1$-$R_4$, A and Z have the meanings given in Claim 1 and X has the meaning given in Claim 3.

6. A method as claimed in Claim 1, characterized in that a compound of formula 13 in which Hal is a halogen atom and $R_4$ is trifluoromethyl is converted with a compound of formula 14 in which $R_1$-$R_3$, A and Z have the meanings given in Claim 1.

7. A method as claimed in Claim 1, characterized in that a compound of formula 15 in which $R_1$-$R_4$, A and Z have the meanings given in Claim 1 is converted with 1,2-dibromoethane.

8. A method as claimed in Claim 1, characterized in that a compound of formula 16 is converted with a compound of formula 17, in which $R_1$-$R_4$, A and Z have the meanings given in Claim 1.

9. A method as claimed in Claim 1, characterized in that a compound of formula 18 is converted with a compound of formula 19, in which $R_1$-$R_4$ and Z have the meanings given in Claim 1 and A is the group $-CH_2-CH_2-$.

10. A method as claimed in Claim 1, characterized in that a compound of formula 9 is converted with a compound of formula 20, in which $R_1$-$R_4$, A and Z have the meanings given in Claim 1.

11. A method as claimed in Claim 1, characterized in that a compound of formula 9 is converted with a compound of formula 12 in the presence of formaldehyde, in which formulae A is the methylene group and $R_1$-$R_4$ and Z have the meanings given in Claim 1.

FORMULA-SHEET

1. 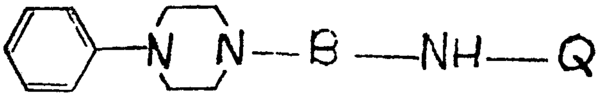

2. 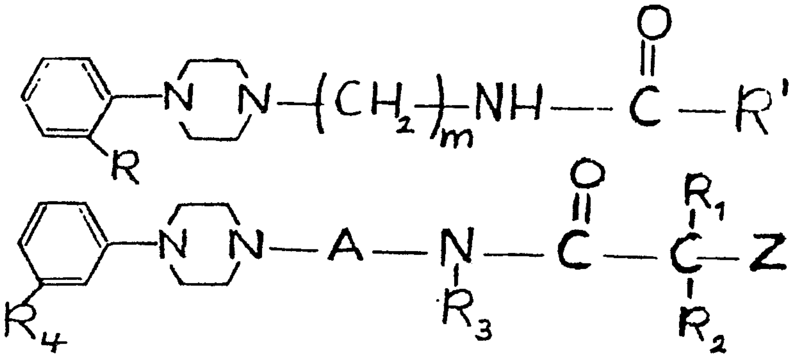

3.

4. 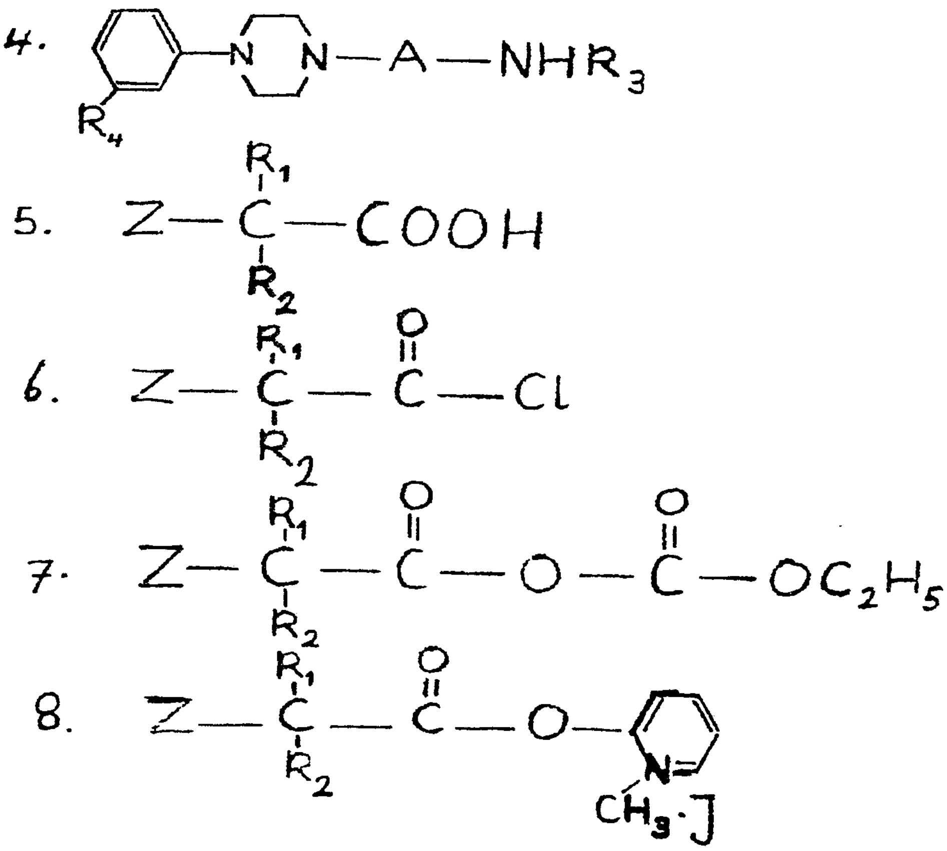

5.

6.

7.

8.

9. 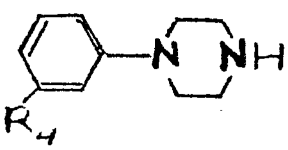

10. 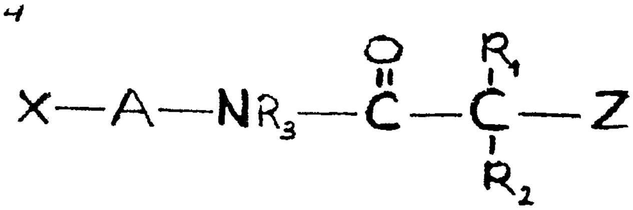

11. 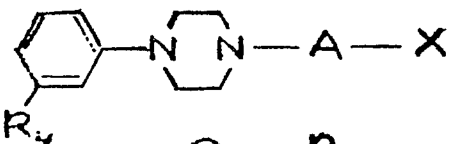

12. 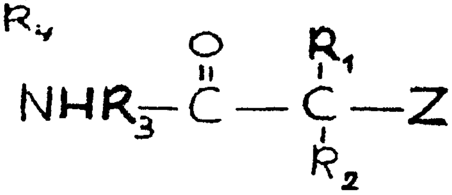

13. 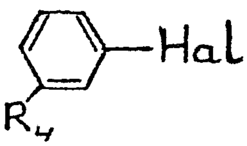

14. 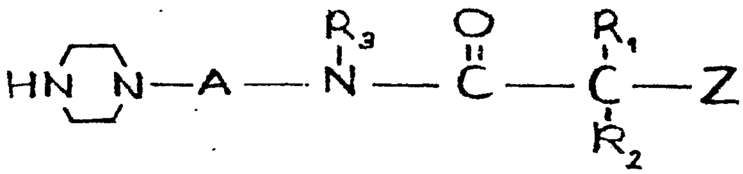

15. 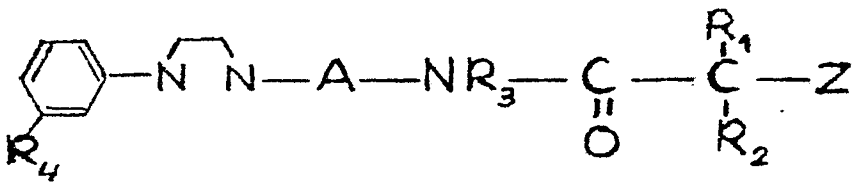

16. 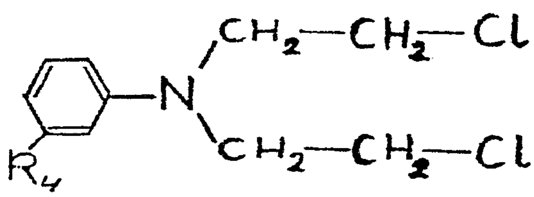

17. 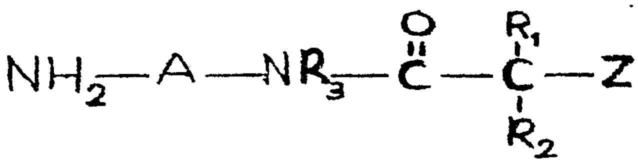

18. 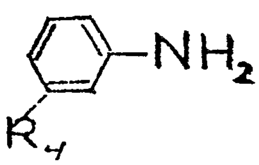

19. 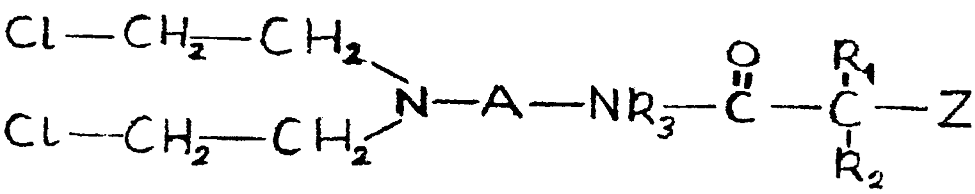

20. 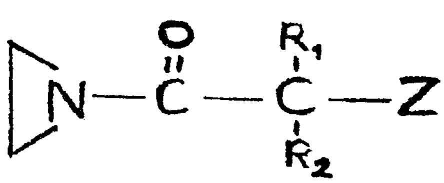

0048045

European Patent Office

# EUROPEAN SEARCH REPORT

Application number
EP 81 20 0929

## DOCUMENTS CONSIDERED TO BE RELEVANT

| Category | Citation of document with indication, where appropriate, of relevant passages | Relevant to claim |
|---|---|---|
| | DE - B - 1 088 058 (PARKE-DAVIS)<br>* Claim *<br>-- | 1 |
| | GB - A - 1 368 256 (PFIZER)<br>* Claims *<br>-- | 1,3-16 |
| | GB - A - 1 166 364 (AMERICAN CY-ANAMID)<br>* Claims *<br>-- | 1,3-16 |
| | AU - A - 55848/80 (published on September 4, 1980)(DUPHAR)<br>& EP - A - 0 015 615<br>---- | 1,3-16 |

CLASSIFICATION OF THE APPLICATION (Int. Cl.3)

C 07 D 295/12
213/56
333/24
A 61 K 31/495

TECHNICAL FIELDS SEARCHED (Int. Cl.3)

C 07 D 295/12
213/56
333/24

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

✓ The present search report has been drawn up for all claims

| Place of search | Date of completion of the search | Examiner |
|---|---|---|
| The Hague | 17-12-1981 | MOREAU |

EPO Form 1503.1 06.78